# EUROPEAN PATENT SPECIFICATION

(11) **EP 4 091 653 B1**
(45) Date of publication and mention of the grant of the patent: **20.11.2024**
(21) Application number: 22382486.3
(22) Date of filing: 20.05.2022
(51) Int. Cl.: A61M 16/00, A61M 16/10

(54) **AUTOMATIC CONTROL SYSTEM FOR MANUAL MECHANICAL VENTILATION DEVICE**
AUTOMATISCHES STEUERUNGSSYSTEM FÜR EINE MANUELLE MECHANISCHE BELÜFTUNGSVORRICHTUNG
SYSTÈME DE COMMANDE AUTOMATIQUE POUR DISPOSITIF DE VENTILATION MÉCANIQUE MANUELLE

(30) Priority: 20.05.2021 EP 21382457
(43) Date of publication of application: 23.11.2022
(73) Proprietor: GPA Medical, S.L., 08030 BARCELONA (ES)
(72) Inventor: ROMAGOSA CALATAYUD, Pau, 08030 BARCELONA (ES); SARSANEDAS MILLET, Pau, 08030 BARCELONA (ES); PEREZ PLANAS, Miguel Francisco, 08030 BARCELONA (ES); BOUFARESS BOUFARESS, Karim, 08030 BARCELONA (ES)
(74) Representative: Herrero & Asociados, S.L.

(56) References cited:
- EP-B1- 0 769 304
- WO-A1-2019/229776
- US-A- 6 155 257
- US-A1- 2019 232 016
- US-A1- 2020 086 075

## Description

### TECHNICAL SECTOR

This invention falls within the medical sector, specifically in the field of respiratory support devices.

### STATE OF THE ART

Respiratory life support devices in hospitals include respirators or mechanical ventilators to supplement lung ventilation.

In situations that overwhelm hospitals, such as epidemics and pandemics, there is a need to make up for the lack of ventilators.

For this reason, several medical devices have recently been developed for mechanical ventilation in emergency situations. Many of these products are based on the automation of a hand-held ventilator (also known by its widely used brand name, Ambu^{®}, from "Airway Mask Bag Unit"). A resuscitator or manual respirator consists of a self-inflating air bladder connected to a mask through a set of valves. When the mask is properly applied and the chamber is manually compressed, the device forces air into the patient's lungs. When manual compression ceases, the chamber self-inflates through the other end with atmospheric or oxygen-enriched air, while allowing the lungs to empty into the environment through the valve assembly. It is a device used in medical vehicles and in transient situations in hospitals. However, they are limited for short periods of time, as they must be manually operated by healthcare personnel. In addition, the manual action required means that constant ventilation is not provided, as parameters such as breaths per minute, total volume applied and inspiration/expiration ratio are difficult to assess subjectively.

Therefore, in the midst of a global pandemic, there is a pressing need to improve the properties of medical devices for emergency mechanical ventilation to bring the ventilation produced in the patient closer to those that would be provided by an advanced medical ventilator.

The following documents are disclosing automatic control systems for ventilation bags, EP 0 769 304 B1, US 6 155 257 A, WO 2019/229776 A1 and US 2020/086075 A1.

### SUMMARY OF THE INVENTION

The invention is defined in independent claim 1. Preferred embodiments are disclosed in the dependent claims.

The object of the present invention is to provide a control system for manual ventilators that allows automatic ventilation to be controlled much more efficiently and accurately than prior art devices, enabling the practitioner to adapt the flow of oxygen and the pressure applied according to the needs of the patient.

To this end, the present disclosure comprises a system for controlling the ventilation produced in the patient by a hand-held ventilator based on the automation of the breathing process. The system is provided for this purpose with a linear actuator with servomotor that regulates the compression of the ventilator and is controlled by a programmable logic controller that receives data from at least one pressure sensor and at least one flow sensor. The system is further provided with an interface for inputting pressure and flow output values. Based on these output values and the sensor readings, the controller acts on the actuator. The components can be connected to each other via a Profinet network and are advantageously equipped with an uninterruptible power supply. A one-way valve, PEEP valve and a humidifying and/or antibacterial filter just before the patient connection can optionally be located in the patient-side socket.

The invention is intended for patients requiring mechanical ventilation or in cardiorespiratory arrest. Its use is limited to medical personnel qualified in the technique of invasive mechanical ventilation.

### BRIEF DESCRIPTION OF THE FIGURES

Figure 1.- is a schematic of the system of the disclosure.
Figure 2.- is a graph of the respiratory cycle and the insufflation period by mechanical ventilation controlled by the system of the disclosure.
Figure 3.- is a particular implementation of the control system applied to an existing ventilator.

### DETAILED DESCRIPTION

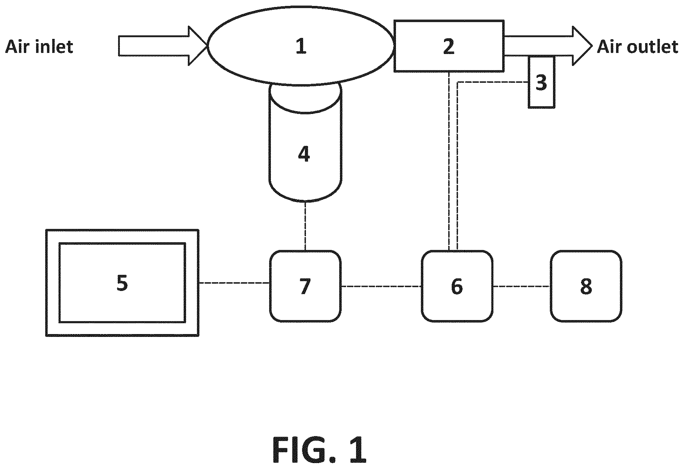

An embodiment of the control system for a hand-held ventilator according to the disclosure is shown in figure 1. The system has an air inlet to the hand-held ventilator and an air outlet from the hand-held ventilator. The control system is equipped with at least one flow sensor and at least one pressure sensor. Signals from these sensors go to the programmable logic controller, which controls the actuator that compresses the ventilation device according to the desired final parameters that the practitioner enters into the system via a human-machine interface based on the sensor readings. The software has to control and communicate with:
- Programmable Logic Controller (PLC)
- Interface
- Actuator
- Pressure sensor
- Flow sensor
- Uninterruptible Power Supply (UPS).

The flow sensor reports and monitors the volume insufflated to the patient in a given time. The pressure sensor controls the pressure ranges applied to the patient. The equipment controls the ventilation applied to the patient by modulating the speed, pressure and stroke that the actuator exerts on the hand-held ventilator.

Using the interface screen, the clinician verifies and modifies the ventilator's operation.

Figure 1 shows an embodiment of a control system for mechanical ventilation based on the automation of a hand-held ventilator (1) according to the disclosure. The system includes a flow sensor (2) and a pressure sensor (3). The compression of the ventilator bag is performed by a servomotor, an actuator (4) and a stepper cylinder. The device has a human-machine interface (5), or HMI, with a display that allows both the input of the necessary parameters for the patient according to the physician and the reading of the information provided by the sensors. An uninterruptible power supply (UPS) shall be used to keep the equipment operational in the absence of external power.

The elements of the system are:
(1) hand-held respirator
(2) flow sensor
(3) pressure sensor
(4) servomotor, actuator and stepper cylinder.
(5) human-machine interface
(6) controller
(7) switch
(8) uninterruptible (continuous) power supply system

The automatic drive part allows the translation of a cylinder step by step by means of a servomotor and actuator associated with the cylinder. The movement of the stepper cylinder in turn allows the hand-held ventilator to be compressed to deliver air to the patient.

The system may include elements to maintain the hand-held ventilator in a correct position for operation. Such elements may include, for example, a set of elastic bands. The system shall have a flow sensor to report and monitor the volume insufflated to the patient. The insufflated volume is calculated by measuring flow and time.

The system will also have a pressure sensor to monitor the pressure ranges applied to the patient.

To control the stepper cylinder, the servo motor actuator communicates directly with the controller, which manages the displacements of the stepper cylinder.

In order to unify the communications of all devices, a Profinet network and a switch shall preferably be used. The switch does not interfere with the other devices as it performs the passive link functions to link the frames/packets between devices. The Profinet industrial network is based on TCP/IP communications but adapted for the exchange of data with industrial devices such as PLC, HMI, Servo drives, etc... where the exchange of packets is done in a fast and deterministic way guaranteeing the quality of service.

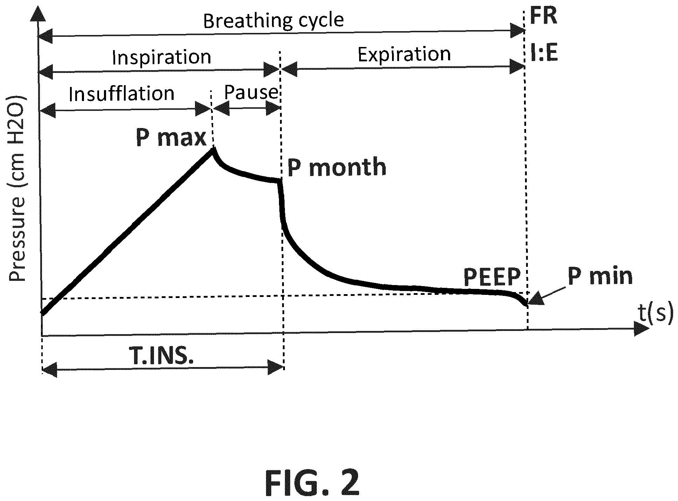

Figure 2 shows a graph of the insufflation periods. These periods depend on the mode of operation of the system:
- Volume control: mandatory ventilatory mode for sedated patients, where the tidal volume is set by the "insufflation" parameter, and respiratory cycles are controlled by respiratory rate and inspiration-expiration (I:E) ratio.
- Volume assisted control: ventilatory support mode for the initial phase of weaning (known as weaning), where the patient has the possibility to set the ventilatory rate. The physician sets the tidal volume using the "insufflation" parameter, sets the baseline respiratory rate and I:E ratio, as well as the inspiratory sensitivity (the effort the patient must make to open the inspiratory valve of the ventilator) to start the respiratory cycle.
- Volume assisted: ventilatory support mode for weaning or weaning, where the patient sets the ventilatory rate. The physician enters the tidal volume using the "insufflation" parameter, sets the inspiratory time and inspiratory sensitivity for the start of the respiratory cycle. In addition, the "apnoea time" can be set, which, if exceeded, the device switches to Volume Control mode.

The system works with the following control parameters:
- Insufflation in % of the volume applied by the hand-held ventilator over the maximum volume of the hand-held ventilator.
- Actual respiratory rate, rpm.
- Inspiration: Expiration Ratio (I:E Ratio).
- Positive end-expiratory pressure (PEEP) in cmH₂ O.
- Insufflation time, in seconds, for Volume Assisted mode
- Inspiratory sensitivity for Volume Control Assisted and Volume Assisted modes

The system receives the following monitoring parameters from the mechanical ventilation equipment:
- Maximum or peak pressure, cmH₂ O
- Plateau pressure, cmH₂ O
- Minimum pressure, cmH₂ O
- Actual respiratory rate, rpm
- Ratio I:E

The information obtained by the sensors can be represented in the form of graphs:
- Pressure cmH₂ O
- Inspiratory flow, L/min
- Tidal volume, mL

The system warns with alarms in different cases:
- Maximum and minimum tidal volume, mL
- Maximum and minimum breathing pressure, cmH₂ O
- Minimum and maximum actual respiratory rate, rpm
- Disconnection of power supply

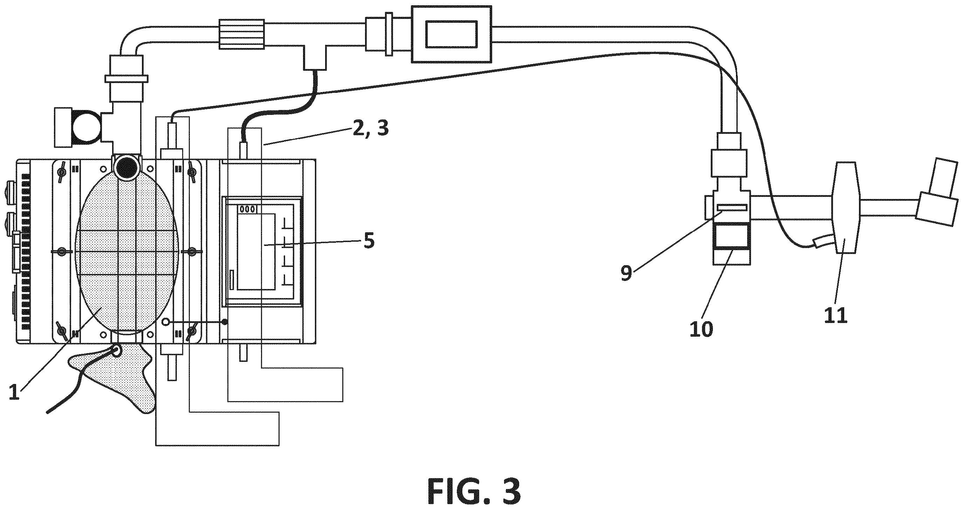

Figure 3 shows some optional elements of the system, in addition to the previously mentioned elements. A one-way valve (9), PEEP valve (10) and a humidifying and/or antibacterial filter (11) can be found in the patient-side tubing just before the patient connection.

In view of this description and figures, the person skilled in the art will understand that the invention has been described according to some preferred embodiments thereof, but that multiple variations may be introduced in said preferred embodiments, without exceeding the subject matter of the invention as claimed.

## Claims

1. Automatic control system for mechanical ventilation of a patient based on the automation of a manual ventilator bag wherein the system comprises a programmable logic controller (6), a stepper cylinder, an actuator and a servomotor associated to the cylinder, (4) such that translation of the cylinder step by step by means of the servomotor and the actuator associated with the cylinder compresses the ventilator bag (1) in response to signals sent by the programmable logic controller (6) wherein said programmable logic controller (6) is connected to the servomotor and to at least one flow sensor (2) and one pressure sensor (3) and has a human-machine interface (5) for the insertion of pressure and oxygen flow output values to be received by a patient, wherein the controller (6) is adapted to modify the compression received by the mechanical ventilation device (2) based on the sensor input and output values entered into the interface (5), **characterised in that** the controller (6) and the sensors are connected via a TCP/IP communication network where the exchange of packets is done in a deterministic way guaranteeing the quality of service, and via a switch (7), wherein the switch performs passive link functions between the devices.

2. Control system according to claim 1, further comprising an uninterruptible power supply system (8).

3. Control system according to any of the preceding claims, wherein the interface is adapted to provide sensor readout.

## Patentansprüche

1. Automatisches Steuerungssystem zur mechanischen Beatmung eines Patienten basierend auf der Automatisierung eines manuellen Beatmungsbeutels, wobei das System eine speicherprogrammierbare Steuerung (6), einen Schrittzylinder, einen Aktuator und einen dem Zylinder zugeordneten Servomotor (4) umfasst, sodass die schrittweise Verschiebung des Zylinders mittels des Servomotors und des dem Zylinder zugeordneten Aktuators den Beatmungsbeutel (1) in Reaktion auf die von der speicherprogrammierbaren Steuerung (6) gesendeten Signale zusammenpresst, wobei die speicherprogrammierbare Steuerung (6) mit dem Servomotor und mit mindestens einem Durchflusssensor (2) und einem Drucksensor (3) verbunden ist und eine Mensch-Maschine-Schnittstelle (5) für die Eingabe von Druck- und Sauerstofffluss-Ausgangswerten aufweist, welche von einem Patienten empfangen werden, wobei die Steuerung (6) dazu ausgelegt ist, die von der mechanischen Beatmungsvorrichtung (2) erhaltene Kompression basierend auf den Sensoreingangsgrößen und den in die Schnittstelle (5) eingegebenen Ausgangswerten zu modifizieren, **dadurch gekennzeichnet, dass** die Steuerung (6) und die Sensoren über ein TCP/IP-Kommunikationsnetzwerk, bei dem der Austausch von Paketen auf deterministische Weise erfolgt, sodass die Dienstleistungsqualität gewährleistet ist, und über einen Switch (7) verbunden sind, wobei der Switch passive Verbindungsfunktionen zwischen den Geräten ausführt.

2. Steuerungssystem nach Anspruch 1, weiter umfassend ein unterbrechungsfreies Stromversorgungssystem (8).

3. Steuerungssystem nach einem der vorangehenden Ansprüche, wobei die Schnittstelle so ausgelegt ist, dass sie eine Sensorauslesung ermöglicht.

## Revendications

1. Système de commande automatique pour la ventilation mécanique d'un patient basé sur l'automatisation d'un ballon de ventilation manuelle, dans lequel le système comprend un automate programmable (6), un cylindre pas à pas, un actionneur et un servomoteur associés au cylindre (4), de sorte que la translation du cylindre pas à pas au moyen du servomoteur et de l'actionneur associés au cylindre comprime le ballon de ventilation (1) en réponse à des signaux envoyés par l'automate programmable (6), dans lequel ledit automate programmable (6) est connecté au servomoteur et à au moins un capteur de débit (2) et un capteur de pression (3) et présente une interface homme-machine (5) pour l'insertion de valeurs de sortie de pression et de débit d'oxygène à recevoir par un patient, dans lequel l'automate (6) est conçu pour modifier la compression reçue par le dispositif de ventilation mécanique (2) sur la base des valeurs d'entrée et de sortie de capteur entrées dans l'interface (5),
**caractérisé en ce que**
l'automate (6) et les capteurs sont connectés par l'intermédiaire d'un réseau de communication TCP/IP où l'échange de paquets s'effectue de manière déterministe, garantissant la qualité de service, et par l'intermédiaire d'un commutateur (7), dans lequel le commutateur assure des fonctions de liaison passive entre les dispositifs.

2. Système de commande selon la revendication 1, comprenant en outre un système d'alimentation électrique sans coupure (8).

3. Système de commande selon l'une quelconque des revendications précédentes, dans lequel l'interface est conçue pour fournir une lecture de capteur.
